# EUROPÄISCHE PATENTANMELDUNG

(11) **EP 0 732 588 A2**
(43) Veröffentlichungstag der Anmeldung: **18.09.1996**
(21) Anmeldenummer: 96104027.6
(22) Anmeldetag: 14.03.1996
(51) Int. Cl.: G01N 33/18, C02F 3/00, C02F 3/12

(54) **Verfahren zur Bestimmung des Kohlenstoffabbaus und der Nitrifikation in biologischen Systemen**

(30) Priorität: 17.03.1995 DE 19509777
(71) Anmelder: GRUNDIG E.M.V. Elektro-Mechanische Versuchsanstalt Max Grundig & Co. KG., D-90762 Fürth (DE)
(72) Erfinder: Hallas, Ernst, Dr., 90762 Fürth (DE)
(74) Vertreter: Niedermeier, Peter

(57) **Zusammenfassung**

Die Erfindung betrifft ein Verfahren zur Bestimmung des Kohlenstoffabbaus und des NH₄⁺-Abbaus in biologischen Systemen, insbesondere in biologischen Abwasserreinigungssystemen, durch die Bestimmung von CO₂ und O₂ in den Ausgasungen an der Oberfläche des biologischen Systems.
Gemäß der Erfindung wird die in das biologische System eingebrachte Konzentration von CO₂ und O₂ bestimmt oder vorgegeben. Die Konzentration von CO₂ und O₂ in der Ausgasung an der Oberfläche des biologischen Systems wird gemessen und die Differenz aus der in das biologische System eingebrachten und der in der Ausgasung bestimmten Konzentration wird gemessen und aus der Sauerstoffzehrung werden die zum O₂-Abbau proportionalen Werte des NH₄⁺-Abbaus und des Kohlenstoffabbaus bestimmt.

## Beschreibung

Die Erfindung betrifft ein Verfahren zur Bestimmung des Kohlenstoffabbaus und der Nitrifikation in biologischen Systemen, insbesondere in biologischen Abwasserreinigungssystemen, gemäß dem Oberbegriff des Anspruches 1.

In Abwasserreinigungsanlagen werden zunächst auf mechanischem Weg die ungelösten Stoffe im Abwasser abgeschieden. Im weiteren werden im Rahmen einer biologischen Reinigung biologisch abbaubare Wasserinhaltsstoffe abgebaut. Voraussetzung für die biologische Abbaubarkeit ist im wesentlichen das Vorhandensein von kohlenstoffhaltigem organischen Material und vor allem von Sauerstoff.

Wegen seiner großen Anpassungsfähigkeit an unterschiedliche Belastungen wird neuerdings überwiegend das Belebtschlammverfahren oder Belebungsverfahren mit unterschiedlichen Mikroorganismen angewandt. Hier schwimmen Bakterienkolonien frei in einem mit dem zu reinigenden Abwasser durchströmten bzw. gefüllten Belebungsbecken. Der zum Abbau der Schadstoffe benötigte Sauerstoff wird beispielsweise mit Belüftern in das Belebungsbecken eingebracht.

Die Belüftung kann sowohl von der Oberfläche her durch Verwirbelung oder aus der Tiefe durch Luft- bzw. Sauerstoffeinblasen erfolgen.

Um die biologische Reinigung effektiver zu gestalten, ist es bekannt, die Sauerstoffverbrauchsrate über die Größe des Sauerstoffüberschusses in Form von gelöstem Sauerstoff mit Partialdrucksonden zu bestimmen und in Abhängigkeit dieser Sauerstoffverbrauchsrate durch definierte Sauerstoffzufuhr die biologischen Prozesse der Abwasserreinigung zu steuern. Dies ist notwendig und zweckmäßig, da in Abwasserreinigungsanlagen sowohl über den Tag als auch über die Woche verteilt Schwankungen in der biologischen Belastung des Abwassers auftreten. Diese Schwankungen werden durch die unterschiedlich starken Abwassereinläufe aus der Industrie und den privaten Haushalten verursacht. So beinhalten die Abwasser beispielsweise während der Nachtstunden oder an Wochenenden nur ein Viertel oder weniger biologisch abbaubare Stoffe als bei Lastspitzen.

Aus der deutschen Offenlegungsschrift DE 42 29 550 A1 ist eine Anordnung zur Regelung der Bioaktivität von biologischen Systemen bekannt, bei dem das in einem Belebtschlammbecken freigesetzte Gas über der Oberfläche des Belebtschlammbeckens in einem oder mehreren Bereichen von der Umgebungsatmosphäre abgeschirmt wird und die Konzentration des Gases innerhalb dieser Abschirmung bestimmt wird. Die Abschirmung, die dort als Gasentnahmesonde dient, ist in Form einer Kuppel ausgebildet, die an schwimmfähigen Elementen angeordnet ist.
Zur Bestimmung der Bioaktivität werden dort die Parameter O₂ oder CO₂ einzeln oder zusammen mittels einer Meßsonde gemessen. Zusätzlich kann dort auch leicht flüchtiger Kohlenwassestoff (HC) gemessen werden, um eine HC-Vergiftung frühzeitig zu erkennen. Durch die Bestimmung des CO₂-Gehaltes in der Ausgasung kann die relative Intensität des Kohlenstoffabbaus erkannt werden.
Da jedoch der Kohlenstoffabbau und der NH₄⁺-Abbau im gleichen biologischen System parallel ablaufen, ergibt sich der Nachteil, daß die Nitrifikation (Ablaufparameter: NH₄⁺) nicht eingeschätzt werden kann. Im weiteren zehrt die Nitrifikation auch Sauerstoff und in geringem Maße CO₂, so daß durch die bekannte Messung keine eindeutigen Aussagen über den Zustand des biologischen Systems zuläßt.

Es ist daher Aufgabe der vorliegenden Erfindung, das bekannte System zur Regelung der Bioaktivität in der Weise zu verbessern, daß alle wichtigen Parameter des biologischen Systems bestimmt werden können.

Diese Aufgabe wird gemäß der Erfindung ausgehend von den Merkmalen des Oberbegriffes des Anspruches 1 durch die kennzeichnenden Merkmale des Anspruches 1 gelöst.

Gemäß der Erfindung wird die in das biologische System eingebrachte Konzentration von CO₂ und O₂ bestimmt oder vorgegeben. Die Konzentration von CO₂ und O₂ in der Ausgasung an der Oberfläche des biologischen Systems wird gemessen und die Differenz aus der in das biologische System eingebrachten und der in der Ausgasung bestimmten Konzentration wird gemessen. Aus der Sauerstoffzehrung werden die zur O₂-Abnahme proportionalen Werte des NH₄⁺-Abbaus und des Kohlenstoffabbaus bestimmt.

Die Vorteile der Erfindung liegen darin, daß der bekannte Sauerstoff und Kohlendioxideintrag in das biologische System mit den in der Ausgasung enthaltenen Konzentrationen verglichen wird und die damit erreichbare Aussage über die Sauerstoffzehrung und den Kohlenstoffabbau zur Bestimmung des NH₄⁺-Abbaus (Nitrifikation) ausgewertet werden.

Es ist bekannt, in welcher Zusammensetzung das Gas für die Belebung in das biologische System eingebracht wird. In der Regel wird als Belebungsgas technisch komprimierte Luft mit der bekannten Zusammensetzung von 78,09 % Stickstoff, 20,95 % Sauerstoff, 0,03 % Kohlendioxid und verschiedenen Edelgasen verwendet. Der Anteil an Sauerstoff und Kohlendioxid beträgt somit ca. 20,98 %.
Stickstoff wird im biologischen System weder produziert noch verbraucht, solange keine Denitrifikation stattfindet.
Zum Abbau von Kohlenstoff wird in Belebungsbecken ca 25 % des verbrauchten Sauerstoffes angesetzt, wobei geringfügige Schwankungen möglich sind. Der verbleibende Rest des verbrauchten Sauerstoffes wird in Belebungsbecken zum größten Teil (ca. 95 %) zur Nitrifikation verbraucht. Bei der Nitrifikation wird NH₄⁺ stufenweise über NO₂⁻ in NO₃⁻ umgewandelt.

Da der Anteil des Sauerstoffverbrauches durch den Kohlenstoffabbau leichten Schwankungen unterworfen ist, hat es sich als vorteilhaft erwiesen, den CO₂-Wert in der Ausgasung zu bestimmen und daraus einen Faktor für die Bewertung des Kohlenstoffabbaus zu bestimmen, da die CO₂-Produktion proportional zum Kohlenstoffabbau ist (ca. 40 % des Kohlenstoffabbaus).

## Patentansprüche

1. Verfahren zur Bestimmung des Kohlenstoffabbaus und des NH₄⁺-Abbaus in biologischen Systemen, insbesondere in biologischen Abwasserreinigungssystemen, durch die Bestimmung von CO₂ und O₂ in den Ausgasungen an der Oberfläche des biologischen Systems,
**dadurch gekennzeichnet, daß**
die in das biologische System eingebrachte Konzentration von CO₂ und O₂ bestimmt oder vorgegeben wird,
die Konzentration von CO₂ und O₂ in der Ausgasung an der Oberfläche des biologischen Systems gemessen wird und die Differenz aus der in das biologische System eingebrachten und der in der Ausgasung bestimmten Konzentration gemessen wird, und
aus der Sauerstoffzehrung die zur O₂-Abnahme proportionalen Werte des NH₄⁺-Abbaus und des Kohlenstoffabbaus bestimmt werden.

2. Verfahren nach Anspruch 1,
**dadurch gekennzeichnet, daß**
aus dem Abbau von CO₂ ein Faktor ermittelt wird, der den Anteil des Kohlenstoffabbaus an der Sauerstoffzehrung gewichtet.
